# EUROPEAN PATENT SPECIFICATION

(11) **EP 4 049 584 B1**
(45) Date of publication and mention of the grant of the patent: **17.07.2024**
(21) Application number: 20878259.9
(22) Date of filing: 22.10.2020
(51) Int. Cl.: A61B 5/11, A61B 5/00, A61B 5/0245, A61B 5/024

(54) **BIOLOGICAL SIGNAL DETECTION DEVICE**
VORRICHTUNG ZUR DETEKTION VON BIOLOGISCHEN SIGNALEN
DISPOSITIF DE DÉTECTION DE SIGNAL BIOLOGIQUE

(30) Priority: 22.10.2019 JP 2019192371
(43) Date of publication of application: 31.08.2022
(73) Proprietor: Delta Tooling Co., Ltd., Hiroshima-shi, Hiroshima 736-0084 (JP)
(72) Inventor: FUJITA, Etsunori, Hiroshima-shi, Hiroshima 736-0084 (JP); KURASHITA, Takashi, Aki-gun, Hiroshima 735-8501 (JP); KAWASAKI, Seiji, Aki-gun, Hiroshima 735-8501 (JP)
(74) Representative: Hoffmann Eitle
(86) International application number: PCT/JP2020/039740
(87) International publication number: WO 2021/079944

(56) References cited:
- WO-A1-2017/172862
- CN-A- 109 008 990
- JP-A- 2003 169 725
- JP-A- 2006 129 933
- JP-A- 2010 069 021
- JP-A- 2013 052 108
- JP-A- 2013 220 810
- JP-A- 2016 026 516
- JP-A- 2016 519 969
- JP-A- 2019 515 708
- US-A1- 2016 367 213
- TSUNENORI TOMIOKA: "Developed by Hiroshima University, a new sensing technology that can detect drivers falling asleep and drinking alcohol", 9 September 2014 (2014-09-09), pages 1 - 5, XP055921333, Retrieved from the Internet <URL:https://xtech.nikkei.com/dm/article/NEWS/20140909/375391>

## Description

### Technical Field

The present invention relates to a biological signal detection device used to detect biological signals used for estimating the conditions of a person.

### Background Art

JP 2013-52108 A discloses a biological signal detection mechanism including a back supporting cushioning member, a base cushioning member, and a sensing mechanism unit disposed between these. This biological signal detection mechanism is attached to a seat back of a seat structure such as a vehicle seat when used, and is used to detect biological signals from the upper body of a person. Arithmetic processing is performed on the detected biological signals, and as is described in JP 2014-117425 A, JP 2014-223271 A and JP 2016-26516 A, the resultants are used as basic data for the determination of a homeostasis function level, the specification of a hypnagogic symptom signal, the estimation of fatigue, and so on. The present inventors have recently further conducted studies aiming at not only using the biological signals for detecting dozing and fatigue during driving but also the application of the biological signals to health care such as blood pressure estimation and health condition estimation.

Another biological signal detection device is disclosed in US 2016/367213 A1 forming the basis for the preamble of claim 1.

### Summary of the Invention

### Problems to Be Solved by the Invention

The biological signal detection mechanism of JP 2013-52108 A has the structure suitable for being attached to the seat back when used as described above, but in some cases, it cannot be said as being always suitable for obtaining biological signals from a person lying on a bed or obtaining biological signals from a part other than the back, for example, from the buttocks, in order to grasp his/her daily health condition, for instance. Further, if the biological signal detection mechanism gives a great feeling of something foreign when obtaining the biological signals from the person in such a lying posture or the like, it may impair comfortable sleeping or sitting.

The present invention was made in consideration of the above and has an object to provide a biological signal detection device that gives only a slight feeling of something foreign to a person, is suitable for detecting biological signals from a person in a lying posture or while placed under the buttocks, and is suitable for detecting biological signals for the purpose of, for example, daily health condition care.

### Means for Solving the Problems

To solve the aforesaid problem, a biological signal detection device of the present invention includes the features of claim 1.

Preferably, a circumferential rim of the body abutting-side cushioning member is compressed and is covered with a band-shaped object.

Preferably, the body abutting-side cushioning member has a diameter equal to or more than the diameter of the base cushioning member.

Preferably, the body abutting-side cushioning member is stacked on the base cushioning member with a slip inhibitor therebetween.

Preferably, in the case where the sensor includes a plurality of sensors, the sensors are provided at equal intervals on the same circumference on the base cushioning member.

### Effect of the Invention

In the biological signal detection device of the present invention, the body abutting-side cushioning member including the three-dimensional knitted fabric is substantially circular in plan view. The base cushioning member is also substantially circular in plan view. If, for example, a person sits on a square one, it tends to undergo deformation such as turning-up or folding of its edges around the vicinity of the corners. Such deformation tends to distort detection data. Moreover, the folding and the like lead to an uncomfortable feeling. On the other hand, the substantially circular one less undergoes such folding and the like. Accordingly, a detection signal obtained from the sensor has only a small distortion and is higher in detection sensitivity than that obtained from the square one. Further, increasing rigidity by compressing the circumferential rim and further covering the circumferential rim with the band-shaped object makes the device fitted to the body and reduces a feeling of something foreign, and owing to the compression of the circumferential rim, surface rigidity of a portion in contact with the body increases. Therefore, the device can be used without impairing comfortable sleeping or comfortable sitting and thus is suitable for measuring biological signals for daily health care.

### Brief Description of Drawings

[FIG. 1] FIG. 1 is a view illustrating the outer appearance of a biological signal detection device of a first example which is not part of the claimed invention.
[FIGs. 2] FIG. 2(a) is a plan view of the biological signal detection device of the first example, FIG. 2(b) is a sectional view taken along its middle, and FIG. 2(c) is its bottom view.
[FIGs. 3] FIG. 3(a) and FIG. 3(b) are plan views of a body abutting-side cushioning member and a base cushioning member, respectively, that are used in the biological signal detection device of the first example.
[FIG. 4] FIG. 4 is an exploded perspective view of the biological signal detection device of the first example.
[FIGs. 5] FIGs. 5 are views of a biological signal detection device according to a second example which is not part of the claimed invention, (a) being its plan view, (b) being its side view, and (c) being a sectional view taken along the C-C line in (a).
[FIG. 6] FIG. 6 is an exploded perspective view of a base cushioning member of the second example.
[FIG. 7] FIG. 7 is a view illustrating the outer appearance of a biological signal detection device of an embodiment of the present invention.
[FIGs. 8] FIG. 8(a) is a plan view of the biological signal detection device of the embodiment, FIG. 8(b) is a sectional view taken along the A-A line in FIG. 8(a), FIG. 8(c) is a sectional view taken along the B-B line in FIG. 8(a).
[FIG. 9] FIG. 9 is a side view of the biological signal detection device of the embodiment.
[FIG. 10] FIG. 10 is an exploded perspective view of the biological signal detection device of the embodiment.
[FIGs. 11] FIG. 11(a) is a view illustrating an example of body pressure distribution when the biological signal detection device according to the first example is placed under the buttocks of a person in a sitting posture and FIG. 11(b) is a view illustrating an example of body pressure distribution when the biological signal detection device according to the third embodiment is placed under a person in a supine posture.
[FIGs. 12] FIG. 12(a) is data of biological signals obtained from the biological signal detection device according to the first example placed under the buttocks and FIG. 12(b) is data of biological signals obtained from a biological signal detection device in a substantially square shape (Comparative Example) placed under the buttocks.
[FIGs. 13] FIGs. 13(a) to (h) are graphs illustrating examples of the analysis results of the data obtained using the biological signal detection device according to the first example.
[FIGs. 14] FIGs. 14(a) to (h) are graphs illustrating other examples of the analysis results of the data obtained using the biological signal detection device according to the first example.
[FIGs. 15] FIGs. 15(a) to (g) are charts illustrating power spectra of the analysis results in FIGs. 13(a) to (e), (g), and (h).
[FIGs. 16] FIGs. 16(a) to (g) are charts illustrating power spectra of the analysis results in FIGs. 14(a) to (e), (g), and (h).

### Modes for Carrying out the Invention

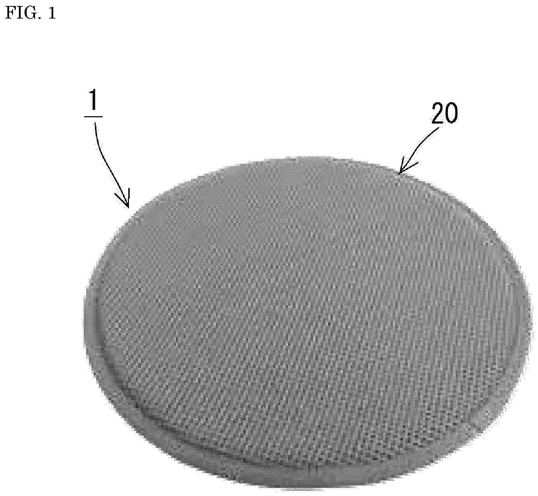

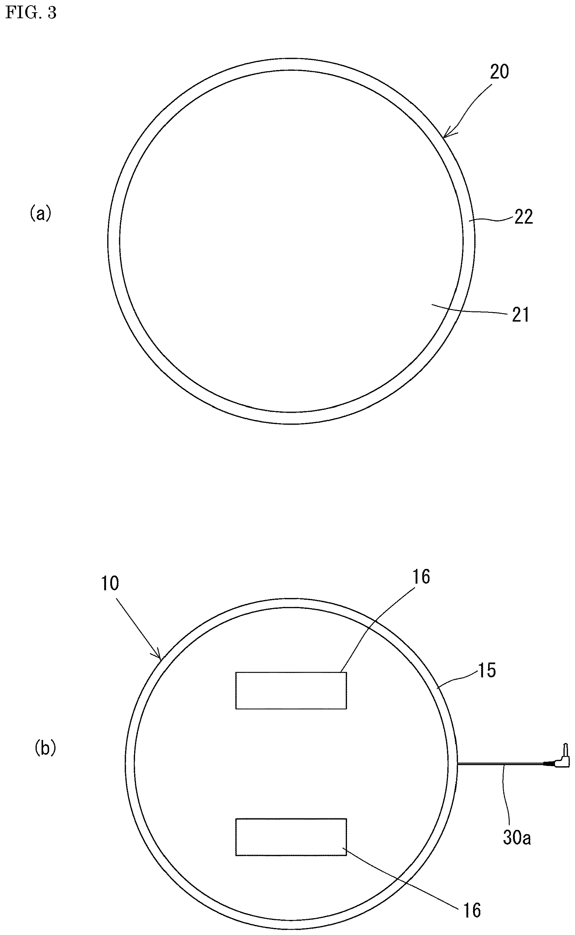

The present invention will be hereinafter described in more detail based on embodiments illustrated in the drawings. FIG. 1 to FIG. 4 are views for explaining a first example. As illustrated in these drawings, a biological signal detection device 1 of this example includes a base cushioning member 10 and a body abutting-side cushioning member 20.

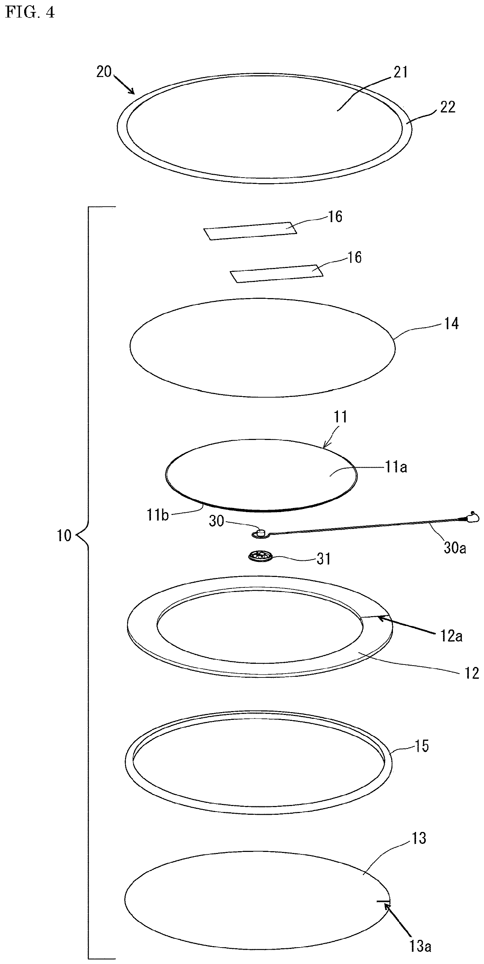

The base cushioning member 10 is substantially circular in plan view, and as illustrated in FIG. 4, includes a substantially circular base-side net 11 having a three-dimensional knitted fabric. The base-side net 11 may be formed of only one three-dimensional knitted fabric but may also be formed of a stack of a plurality of three-dimensional knitted fabrics or a stack of the three-dimensional knitted fabric and a two-dimensional fabric, synthetic leather, or the like. Around the base-side net 11, a ring-shaped bead foam 12 formed of polyethylene foam or the like is disposed. A sensor 30 is disposed on a rear surface 11b of the base-side net 11. The sensor 30 is covered with an elastomer film 13 having a diameter of the combination of the base-side net 11 and the ring-shaped bead foam 12 disposed therearound. The elastomer film 13 is bonded to the ring-shaped bead foam 12.

Similarly to the above, an elastomer film 14 substantially equal in diameter to the bead foam 12 is disposed on a front surface 11a of the base-side net 11 and is bonded to the bead foam 12. The two elastomer films 13, 14 thus sandwich the base-side net 11, the bead foam 12, and the sensor 30, and their circumferential rims are covered with a band-shaped object 15 made of a synthetic resin such as polypropylene.

As the sensor 30, a microphone is used, and preferably an electrostatic microphone is used. In this example, the single sensor 30 is used. In the case where the single sensor 30 is disposed in the biological signal detection device 1, it is preferably provided at the center portion of the substantially circular base-side net 11 so that it can collect biological signals in whichever direction a person moves. The sensor 30 is disposed between the base-side net 11 and the elastomer film 13 with a holder 31 between the sensor 30 and the elastomer film 13. In the ring-shaped bead foam 12 and the elastomer film 13, incisions 12a, 13a are formed near their outer peripheries, and a cord 30a of the sensor 30 is drawn out through the incisions 12a, 13a.

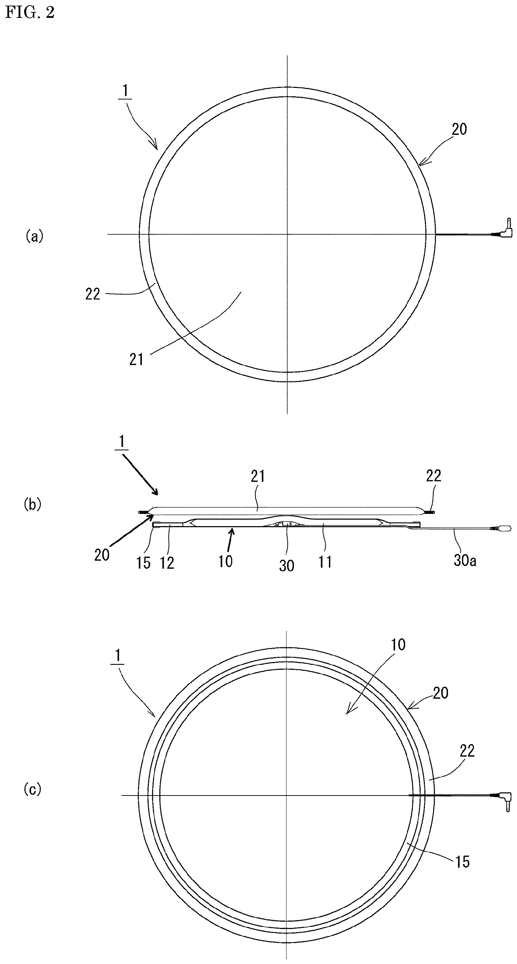

The body abutting-side cushioning member 20 is substantially circular in plan view and includes a substantially circular body abutting-side net 21 having a three-dimensional knitted fabric. The body abutting-side net 21 may be formed of only one three-dimensional knitted fabric but may also be formed of a stack of a plurality of three-dimensional knitted fabrics or a stack of the three-dimensional knitted fabric and a two-dimensional fabric, synthetic leather, or the like. The circumferential rim of the body abutting-side net 21 is covered with a band-shaped object 22 made of a synthetic resin such as polypropylene. As illustrated in the sectional view in FIG. 2(b), the circumferential rim is compressed in the thickness direction and the band-shaped object 22 is attached thereto by sewing. The band-shaped object 22 prevents the fraying of the three-dimensional knitted fabric forming the body abutting-side net 21, and besides, the thinning of the thickness of the circumferential rim increases rigidity, makes the body abutting-side cushioning member 20 easily fitted to the body, and reduces a level difference from the surface of a bed, a seat surface of a chair, or the like where the biological signal detection device 1 is placed, to alleviate a feeling of something foreign when the biological signal detection device 1 is used. Because of the substantially circular shape, the compressing of the circumferential rim increases surface rigidity of a part on the inner side of the circumferential rim substantially uniformly, and alleviates an uncomfortable feeling when the body abutting-side cushioning member 20 abuts on the body. If a person feels pain such as lower back pain at the time of the measurement of biological signals, the detection of the cause of a disease such as a heart disease that should be detected is hindered, but the body abutting-side cushioning member 20 of this exampleonly slightly gives an uncomfortable feeling when abutting on the body as described above, and for example, in the case where it is placed near the lower back of a person in a lying posture, it does not cause lower back pain and thus is suitable for the detection of biological signals.

The body abutting-side cushioning member 20 is stacked on the base cushioning member 10 with slip inhibitors 16 therebetween. The slip inhibitors 16 are formed of grid-shaped sheets made of vinyl chloride resin foam and are attached by lamination to the elastomer film 14 stacked on the front surface 11a of the base-side net 11. The body abutting-side net 21 may be formed of the three-dimensional knitted fabric and besides may be one in which a synthetic leather or the like is stacked thereon as described above, but since this example is configured to reduce the displacement of the body abutting-side cushioning member 20 using the slip inhibitors 16, the body abutting-side net 21 is preferably formed of a stack of the three-dimensional knitted fabric and a synthetic leather, another plastic film, or the like stacked on a rear surface of the three-dimensional knitted fabric.

Instead of fixing the body abutting-side cushioning member 20 to the base cushioning member 10 by sewing, bonding, or the like to prevent their relative movement, the slip inhibitors 16 are simply interposed therebetween as described above to make the displacement of the body abutting-side cushioning member 20 difficult while allowing its slight movement. In the case where they are substantially integrated by sewing or the like, if the body abutting-side cushioning member 20 is moved to be deformed by body movement, the base cushioning member 10 also moves together. This causes the sensor 30 to greatly move together or to greatly change in its placement posture, which may affect detection sensitivity. On the other hand, in the case where the slip inhibitors 16 are used, the body abutting-side cushioning member 20 whose slight movement is allowed easily follows the body movement, but the base cushioning member 10 does not move greatly even though trying to move with the movement of the body abutting-side cushioning member 20, and more tends to keep its posture than in the case where they are firmly fixed by sewing or the like. On the other hand, without the slip inhibitors 16, the body movement or the like may cause the body abutting-side cushioning member 20 to easily separate from the base cushioning member 10. Therefore, it is preferable to stack the body abutting-side cushioning member 20 on the base cushioning member 10 while interposing therebetween the slip inhibitors 16 that allow a slight movement of the body abutting-side cushioning member 20 but make the body abutting-side cushioning member 20 difficult to deviate from the base cushioning member 10.

The body abutting-side cushioning member 20 preferably has a diameter equal to or more than the diameter of the base cushioning member 10. The body abutting-side net 21 having the three-dimensional knitted fabric and forming the body abutting-side cushioning member 20 has a function of alleviating a feeling of contact with a person owing to the deflection of connecting yarns and so on of the three-dimensional knitted fabric. Therefore, the body abutting-side cushioning member 20 preferably has the aforesaid size so that it is capable of covering the entire base cushioning member 10 as much as possible even if the displacement occurs.

The biological signal detection device 1 of this example is placed on, for example, a chair or a bed with the base cushioning member 10 on the lower side. Therefore, a person seats or lays himself/herself on the body abutting-side cushioning member 20. Consequently, biological signals of the person are transmitted to the body abutting-side cushioning member 20 through the body surface. Having a predetermined cushioning property, the body abutting-side cushioning member 20 does not easily give an uncomfortable feeling because the weight of the person is dispersed therein. Further, since its circumferential rim is compressed and is covered with the band-shaped object 22 as described above, a level difference from the seat surface of the chair or the other part of the bed is small, and the person does not easily have a feeling of something foreign even if the body abutting-side cushioning member 20 is slightly displaced by the body movement.

Since the body abutting-side cushioning member 20 has the body abutting-side net 21 having the three-dimensional knitted fabric, biological signals transmitted as sound/vibration information are propagated to the ground knitted fabrics and the connecting yarns forming the three-dimensional knitted fabric and are transmitted into the base-side net 11 having the three-dimensional knitted fabric, which is sandwiched by the elastomer films 13, 14 of the base-side net 11 and has the periphery surrounded by the bead foam 12. The sound/vibration information is collected by the sensor 30, and as is described in the section of Background Art, is used as data for analyses by a smartphone, a laptop personal computer, or other information processing device that estimates sleepiness, fatigue, blood pressure, health condition, and so on. Therefore, the measurement is easily done using the biological signal detection device 1 of this example not only in professional facilities such as hospitals but also in offices and homes, and it is possible to easily practice daily health care only by installing specialized software in a smartphone or the like.

According to this example, the base cushioning member 10 and the body abutting-side cushioning member 20 are both substantially circular in plan view. Unlike rectangular or other polygonal ones, they scarcely undergo the turning-up or folding of their circumferential rims caused by the application of the body weight when, for example, a person seats himself/herself. Theretofore, detection signals of the sensor 30 have less distortion than in the case where they are rectangular or the like, leading to high detection accuracy of biological signals.

The three-dimensional knitted fabrics used in the base-side net 11 and the body abutting-side net 21 are each a knitted fabric with a three-dimensional structure having a pair of ground knitted fabrics disposed apart from each other and many connecting yarns reciprocating between the pair of ground knitted fabrics to connect them as is disclosed in, for example, JP 2002-331603 A. One of the ground knitted fabrics is formed to have, for example, a flat knitted fabric structure (fine meshes) continuous both in a wale direction and a course direction using yarns of twisted monofilaments, and the other ground knitted fabric is formed to have, for example, a mesh structure having honeycomb (hexagonal) meshes using yarns of twisted staple fibers. The knitted fabric structures may of course be any, and their combination may also be any, for example, they may adopt a knitted fabric structure other than the fine mesh structure and the honeycomb structure, or they may both adopt the fine mesh structure. The connecting yarns are knitted between the two ground knitted fabrics so that one of the ground knitted fabrics and the other ground knitted fabric are kept at a predetermined interval. By adjusting the mesh density of the ground knitted fabrics, the thickness or material of ground yarns, the arrangement density of the connecting yarns, the thickness or material of the connecting yarns, and so on, it is possible to adjust the cushioning property when a person is supported and the transmissibility of the biological signals which are the sound/vibration information.

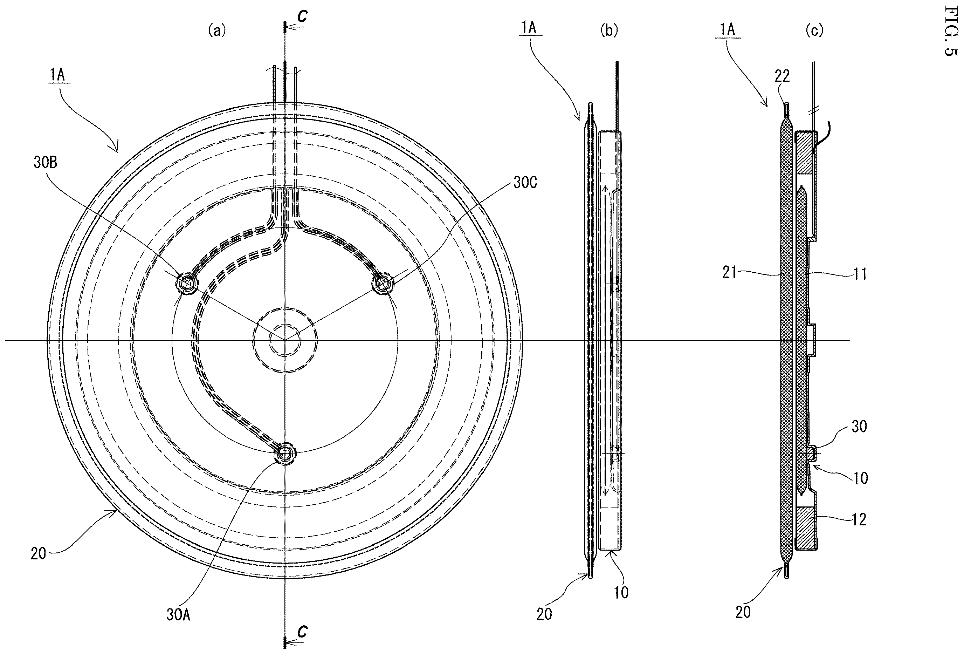

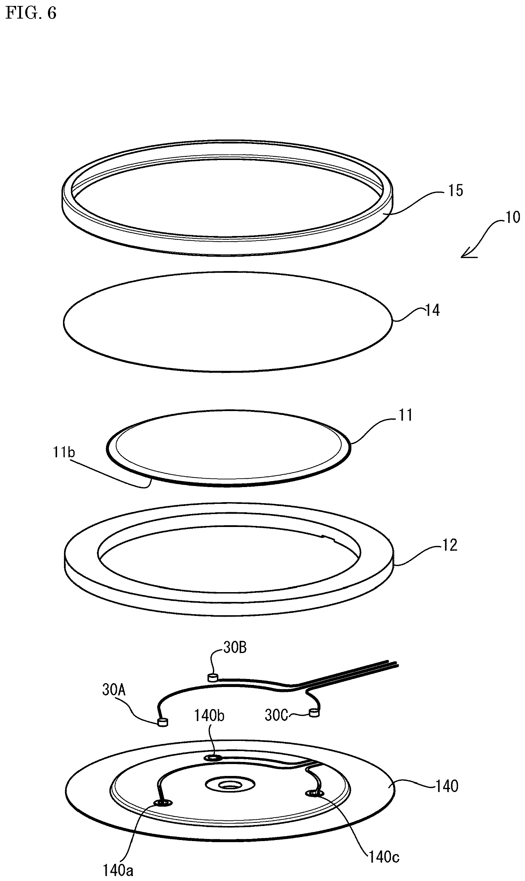

FIGs. 5 and FIG. 6 are views of a biological signal detection device 1A according to a second example . In this example, three sensors 30A, 30B, 30C are disposed on a base cushioning member 10. The three sensors 30A, 30B, 30C are each constituted by a microphone as in the above. Further, the three sensors 30A, 30B, 30C are arranged at equal intervals on the same circumference on the base cushioning member 10. In order to arrange the three sensors 30A, 30B, 30C at equal intervals on the same circumference, specifically, in order to arrange them such that lines connecting the centers of the three sensors 30A, 30B, 30C form an equilateral triangle in plan view, a sensor setting plate 140 having three sensor support portions 140a, 140b, 140c arranged at equal intervals on the same circumference is disposed on a rear surface 11b side of a base-side net 11. Specifically, by placing the sensors 30A, 30B, 30C on the respective sensor support portions 140a, 140b, 140c of the sensor setting plate 140, it is possible to easily arrange the sensors 30A, 30B, 30C at equal intervals on the same circumference. The other structure is substantially the same as that of the above-described first example, and the same members will be denoted by the same reference signs.

With the above-described structure, even if a body abutting-side cushioning member 20 is moved by body movement, any one of the sensors 30A, 30B, 30C is capable of detecting a biological signal. Further, if biological signals are obtained from the plurality of sensors 30A, 30B, 30C, high-sensitivity data out of these is used or a combination of the plurality of biological signals is used, which more widens a data analysis method. It should be noted that the number of the sensors is not limited to three and may be two, or four or more. In this case as well, they are preferably arranged at equal intervals on the same circumference as in this example.

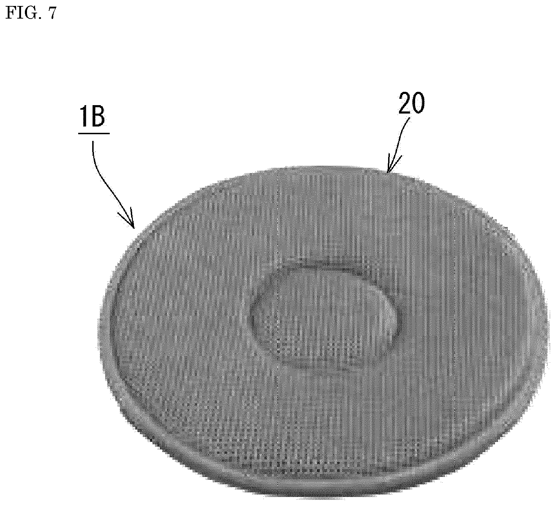

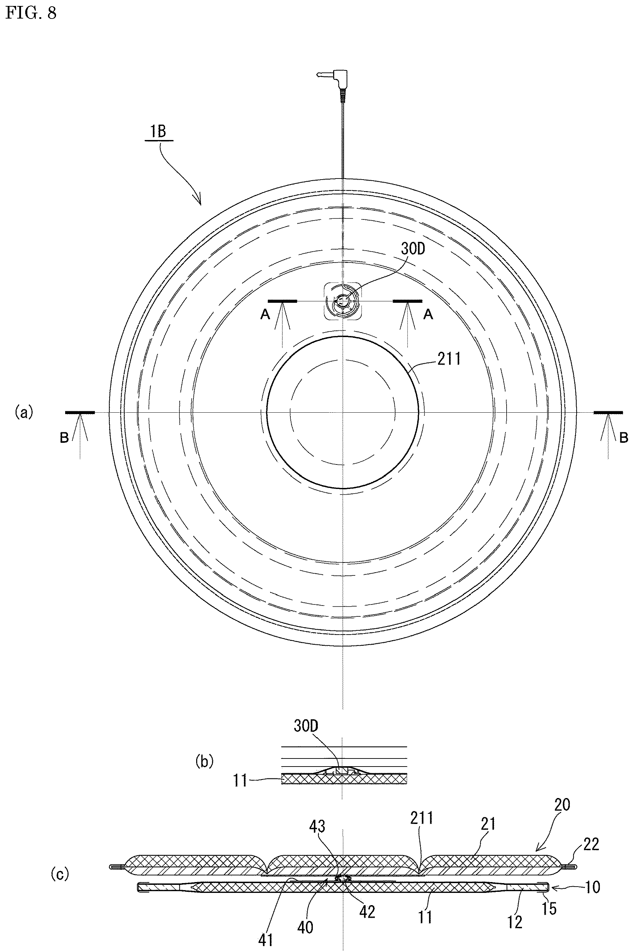

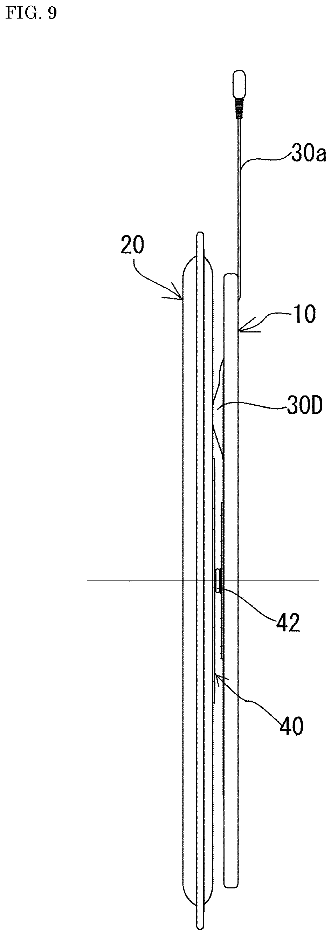

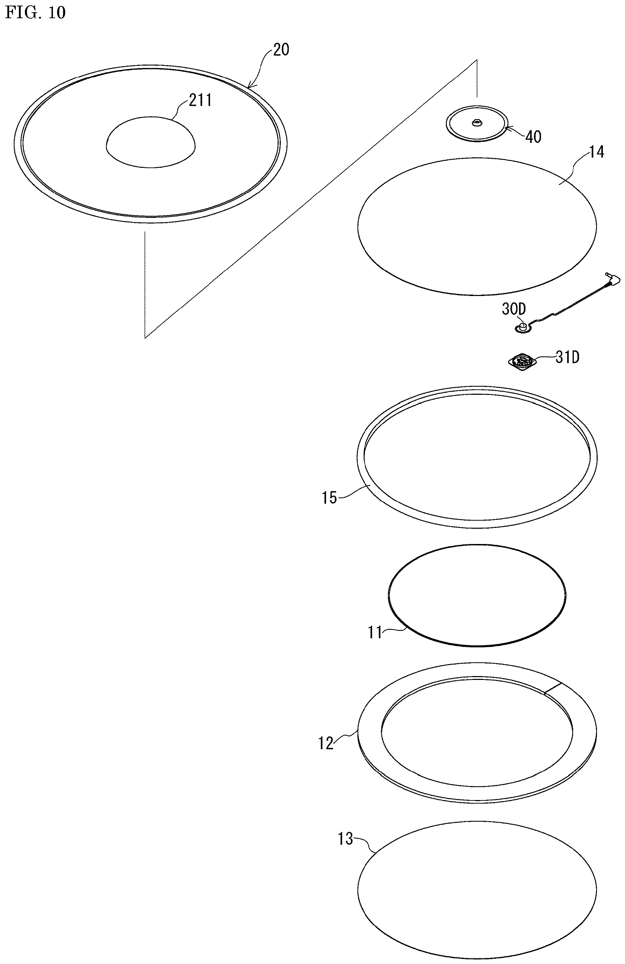

FIG. 7 to FIG. 10 are views of a biological signal detection device 1B according to an embodiment of the present invention. In this embodiment, a shaft member 40 is provided between center portions of facing surfaces of a base cushioning member 10 and a body abutting-side cushioning member 20. The shaft member 40 has a circular plate member 41 provided on the base cushioning member 10 side, a shaft portion 42 located at the center of the plate member 41, and a bearing portion 43 which is provided on the rear surface side of the body abutting-side cushioning member 20 and to which the shaft portion 42 is fitted. With this structure, the body abutting-side cushioning member 20 is rotatable around the shaft portion 42 relative to the base cushioning member 10.

Since the shaft member 40 is provided between the base cushioning member 10 and the body abutting-side cushioning member 20, in the body abutting-side cushioning member 20, a circumferential sewing line 211 is formed by sewing a portion apart slightly outward from the center (portion corresponding to the vicinity of the outer edge of the aforesaid circular plate member 41) in the circumferential direction, to compress a body abutting-side net 21. Owing to the circumferential sewing line 211, the tension of the three-dimensional knitted fabric in a range surrounded by the circumferential sewing line 211 is higher than in a state without the circumferential sewing line 211 formed. This prevents the shaft member 40 from giving a feeling of something foreign.

Since the shaft member 40 is disposed at the center portion, a sensor 30D is disposed using a holder 31D at a position corresponding to a slightly more outer side than the circumferential sewing line 211 in plan view. In the case where only the single sensor is provided as in the above-described first example, it is preferably provided at the center portion of the base cushioning member 10, but in the case where such a shaft member 40 is disposed, the sensor is provided in a range clear of the shaft member 40. In this case as well, by providing a plurality of sensors, preferably providing a plurality of sensors at equal intervals on the same circumference as in the above-described second example, it is possible to increase detection accuracy. Further, in this embodiment, the sensor D is disposed between a base-side net 11 and an elastomer film 14 stacked on a front surface 11a of the base-side net 11. The other structure is substantially the same as that of the above-described first and second examples, and the same members will be denoted by the same reference signs.

According to this embodiment, the body abutting-side cushioning member 20 is not readily displaced relative to the base cushioning member 10 in a direction in which it slides in a plane direction. Accordingly, the position of the body abutting-side cushioning member 20 does not greatly deviate from the position of the base cushioning member 10. On the other hand, since the shaft member 40 is provided, the body abutting-side cushioning member 20 moves in the rotation direction around the shaft portion 42 when body movement occurs. Therefore, the followability to the body movement is not impaired and an uncomfortable feeling given to a user is reduced.

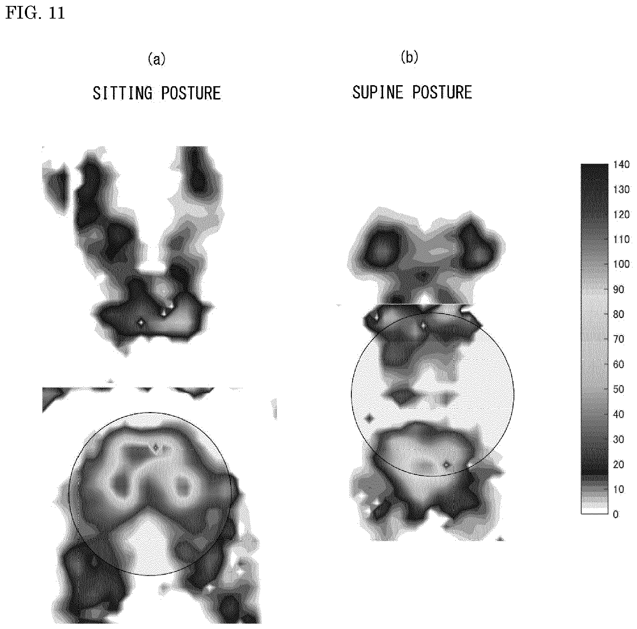

Here, FIG. 11(a) illustrates body pressure distribution when the biological signal detection device 1 according to the first example in FIG. 1 to FIG. 4 is placed under the buttocks of a person in a sitting posture. In the drawing, the part surrounded by the circle is the position of the biological signal detection device 1. As is illustrated in the drawing, the pressure is slightly high near parts under the ischial tuberosities, which shows that it is possible to detect biological signals by placing the biological signal detection device 1 under the buttocks. Further, FIG. 11(b) illustrates body pressure distribution when the biological signal detection device 1B according to the embodiment in FIG. 7 to FIG. 10 is placed under a person in a supine posture. As is seen in the circle-surrounded part where the biological signal detection device 1B is placed, the body pressure is small near the lower back, and thus the device causes lower back pain only a little even placed under the person in the supine posture, and contrarily, the pressure is slightly high near the buttocks and thus it is possible to detect biological signals from a part around this position.

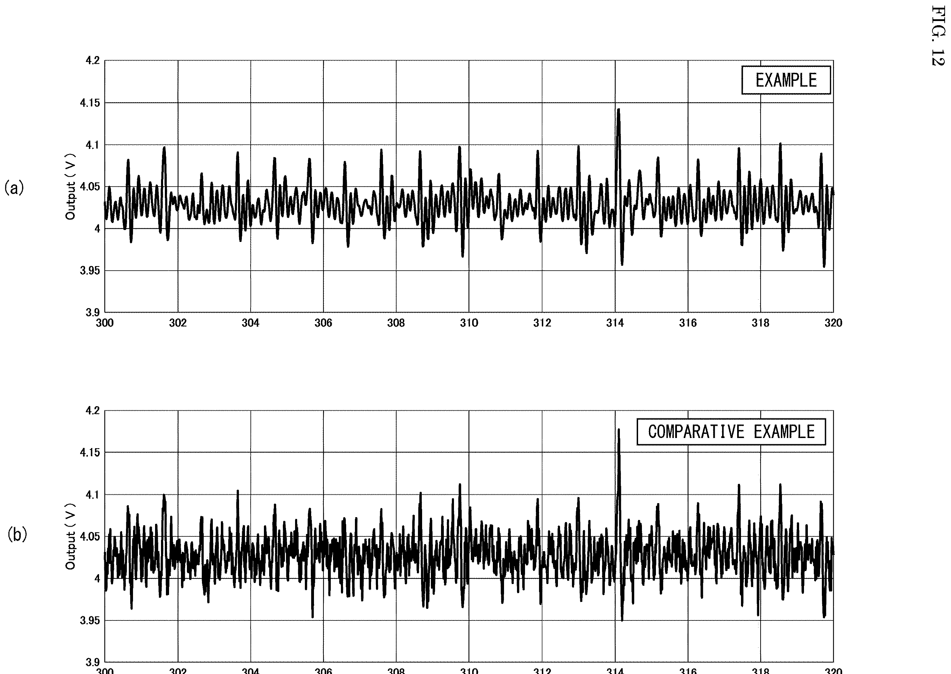

Further, FIG. 12(a) is data of biological signals obtained from the biological signal detection device 1 (Example) according to the first example in FIG. 1 to FIG. 4 placed under the buttocks. FIG. 12(b) is data obtained from a biological signal detection device (Comparative Example) having a substantially equal area to that of the biological signal detection device 1 according to the first embodiment but having a substantially square shape when it is placed under the buttocks of the same subject. Comparison of the two data shows that the data of Comparative Example contains a lot of noise. Therefore, the substantially circular structure in plan view as in the present invention is suitable for capturing a weak biological signal more accurately.

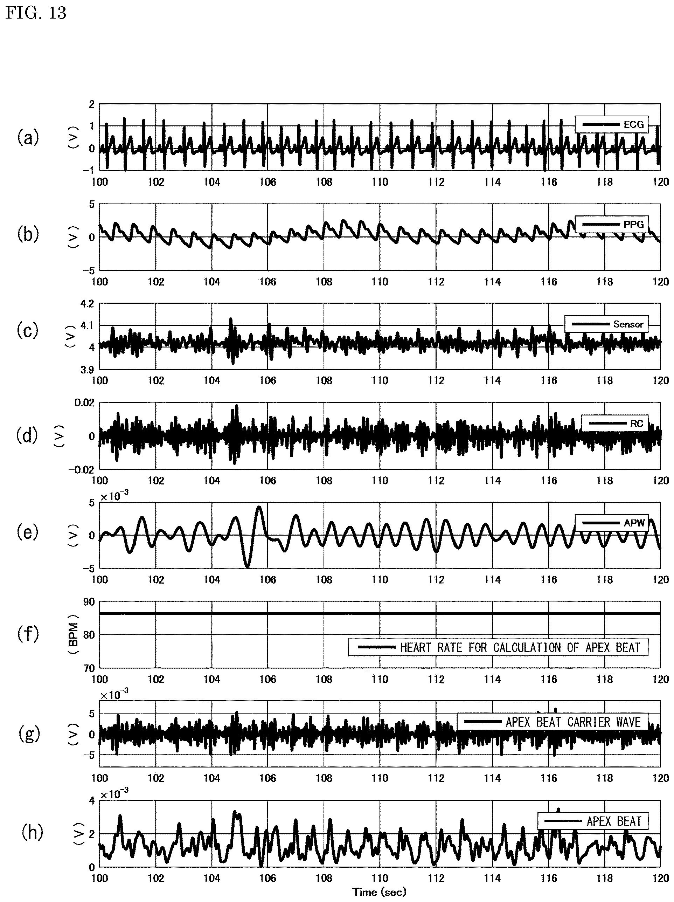

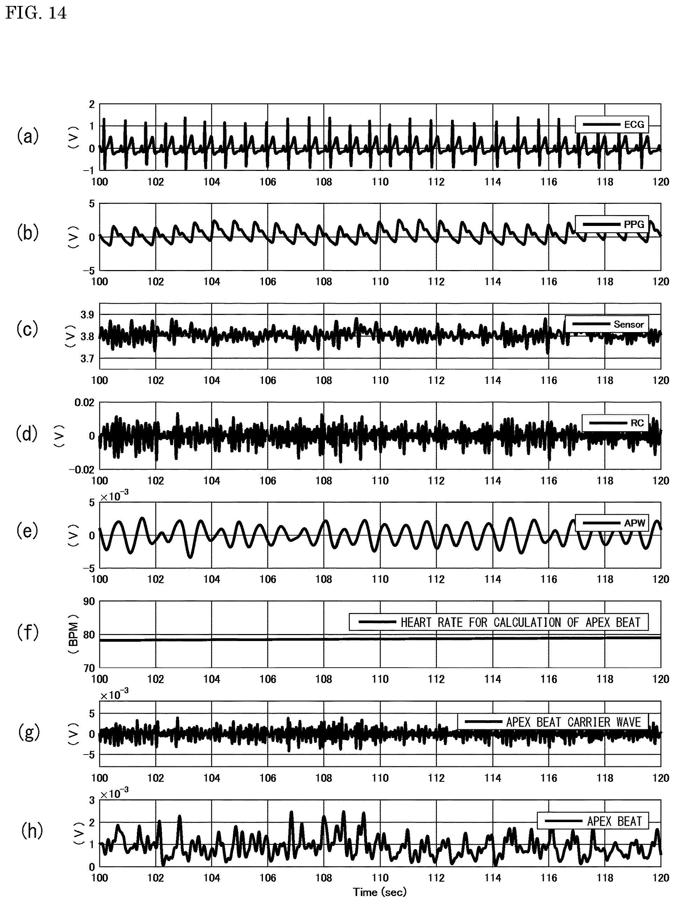

FIGs. 13 and FIGs. 14 are charts illustrating data of biological signals obtained from the biological signal detection device 1 according to the first example in FIG. 1 to FIG. 4 placed near the buttocks (FIGs. 13) and near the lower back (FIG. 14) of the same subject in the supine posture, and examples of analyses using these data. Out of FIGs. 13(a) to (h) and FIGs. 14(a) to (h), (a) illustrate data of electrocardiograms measured at the same time, and (b) illustrate data of finger plethysmograms also measured at the same time. (c) illustrate the data obtained from the biological signal detection device 1, (d) waveforms resulting from filtering of the data in (c), (e) aortic pulse waves (APW) with approximately 1 Hz found through processing of (d), (f) heart rates, (g) apex beat carrier waves, and (h) apex beats.

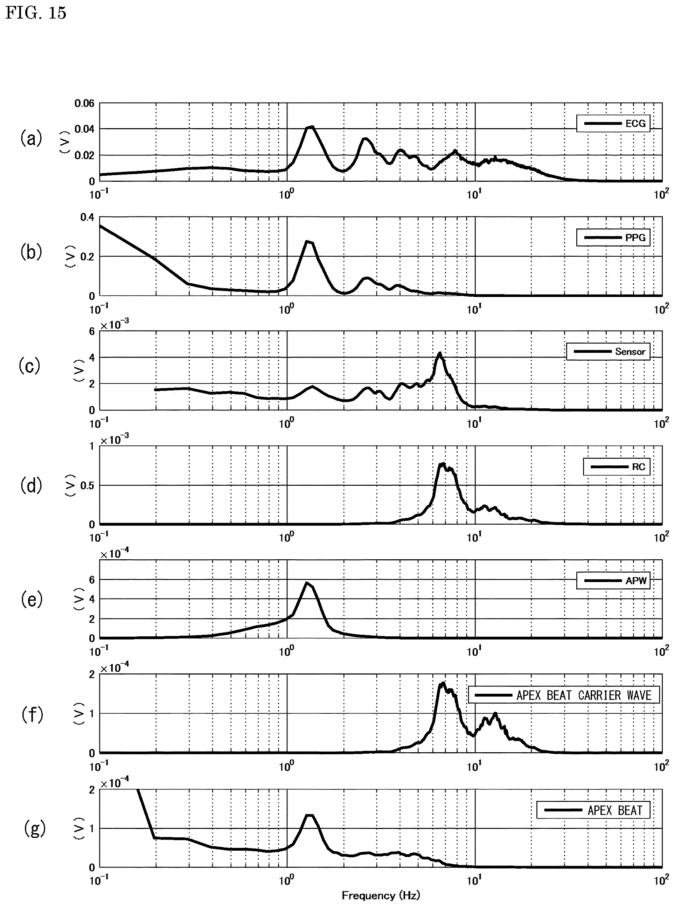

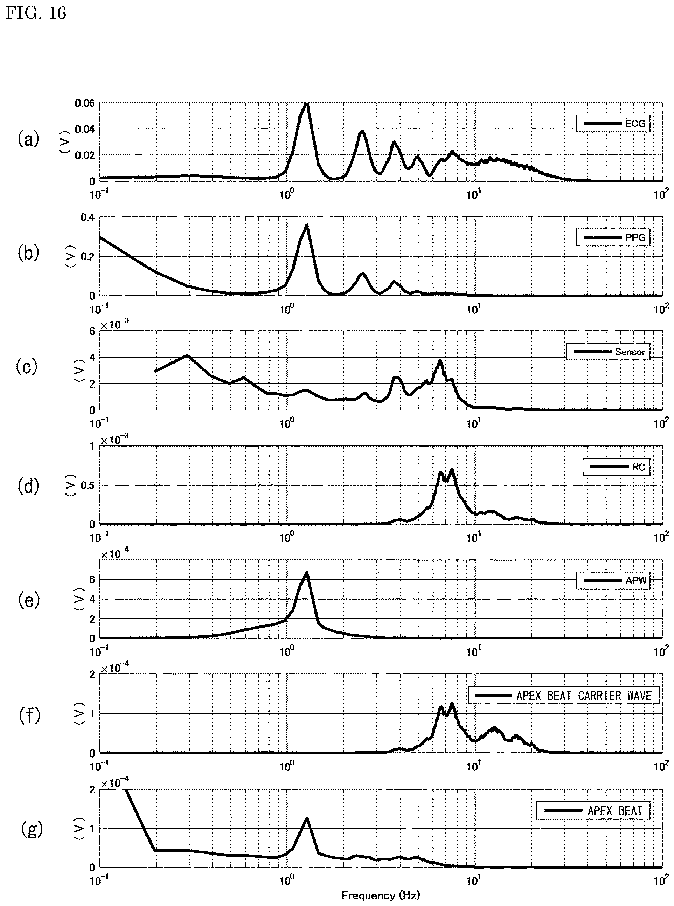

FIGs. 15 and FIGs. 16 are power spectra found regarding the data in FIGs. 13 and FIGs. 14 except the heart rates. Out of FIGs. 15(a) to (g) and FIGs. 16(a) to (g), (a) illustrate power spectra of the electrocardiograms, (b) of the finger plethysmograms, and (c) of the data obtained from the biological signal detection device 1, and (d) of the waveforms resulting from the filtering of the data of (c), (e) of the aortic pulse waves (APW) with approximately 1 Hz found through the processing of (d), (f) of the apex beat carrier waves, and (g) of the apex beats.

As is seen from FIGs. 13 to FIGs. 16, the use (no use by itself is falling within the scope of the claims) of the data obtained from the biological signal detection device 1 according to the above-described example enables the analysis of the biological conditions of a person.

### Explanation of Reference Signs

- 1, 1A, 1B: biological signal detection device
- 10: base cushioning member
- 11: base-side net
- 12: bead foam
- 13, 14: elastomer film
- 16: slip inhibitor
- 20: body abutting-side cushioning member
- 21: body abutting-side net
- 211: circumferential sewing line
- 22: band-shaped object
- 30, 30A, 30B, 30C, 30D: sensor
- 40: shaft member

## Claims

1. A biological signal detection device (1A, 1B) comprising:
a base cushioning member (10) including: a base-side net (11) having a three-dimensional knitted fabric; and a sensor (30A, 30B, 30C, 30D) disposed on one surface or another surface of the base-side net (11); and
a body abutting-side cushioning member (20) stacked on the base cushioning member (10) and including a body abutting-side net (21) which has a three-dimensional knitted fabric and is disposed on a side abutting on a body of a person,
**characterized in that** the base cushioning member (10) and the body abutting-side cushioning member (20) are both substantially circular in plan view,
wherein the body abutting-side cushioning member (20) is stacked on the base cushioning member (10) with a shaft member (40) provided between center portions of the body abutting-side cushioning member (20) and the base cushioning member (10), and
wherein the sensor (30A, 30B, 30C, 30D) is disposed in a range clear of the shaft member (40).

2. The biological signal detection device (1A, 1B) according to claim 1, wherein a circumferential rim of the body abutting-side cushioning member (20) is compressed and is covered with a band-shaped object (22).

3. The biological signal detection device (1A, 1B) according to claim 1 or 2, wherein the body abutting-side cushioning member (20) has a diameter equal to or more than the diameter of the base cushioning member (10).

4. The biological signal detection device (1A, 1B) according to any one of claims 1 to 3, wherein the body abutting-side cushioning member (20) is stacked on the base cushioning member (10) with a slip inhibitor (16) therebetween.

5. The biological signal detection device (1A) according to any one of claims 1 to 4, wherein the sensor (30A, 30B, 30C) includes a plurality of sensors provided at equal intervals on a same circumference on the base cushioning member (10).

6. The biological signal detection device (1A) according to any one of claims 1 to 5, wherein a circumferential sewing line (211) is formed by sewing a portion apart slightly outward from the center in the circumferential direction, to compress a body abutting-side net (21), wherein sensor (30A, 30B, 30C, 30D) is disposed using a holder (31A, 31B, 31C, 31D) at a position corresponding to a more outer side than the circumferential sewing line (211) in plan view.

## Patentansprüche

1. Vorrichtung zur Detektion von biologischen Signalen (1A, 1B), umfassend:
ein Basispolsterelement (10), einschließend: ein basisseitiges Netz (11), das ein dreidimensionales Strickgewebe aufweist; und einen Sensor (30A, 30B, 30C, 30D), der auf einer Oberfläche oder einer anderen Oberfläche des basisseitigen Netzes (11) angeordnet ist; und
ein körperseitig anliegendes Polsterelement (20), das auf dem Basispolsterelement (10) gestapelt ist, und ein körperseitig anliegendes Netz (21) einschließt, das ein dreidimensionales Strickgewebe aufweist und an einer an einen Körper einer Person anliegenden Seite angeordnet ist,
**dadurch gekennzeichnet, dass** das Basispolsterelement (10) und das körperseitig anliegende Polsterelement (20) in einer Draufsicht beide im Wesentlichen kreisförmig sind,
wobei das körperseitig anliegende Polsterelement (20) auf dem Basispolsterelement (10) gestapelt ist, wobei ein Schaftelement (40) zwischen Mittelabschnitten des körperseitig anliegenden Polsterelements (20) und des Basispolsterelements (10) vorgesehen ist, und
wobei der Sensor (30A, 30B, 30C, 30D) in einem Bereich angeordnet ist, der vom Schaftelement (40) frei liegt.

2. Vorrichtung zur Detektion von biologischen Signalen (1A, 1B) nach Anspruch 1, wobei ein umlaufender Rand des körperseitig anliegenden Polsterelements (20) zusammengedrückt und mit einem bandförmigen Objekt (22) bedeckt ist.

3. Vorrichtung zur Detektion von biologischen Signalen (1A, 1B) nach Anspruch 1 oder 2, wobei das körperseitig anliegende Polsterelement (20) einen Durchmesser aufweist, der gleich oder größer ist als der Durchmesser des Basispolsterelements (10).

4. Vorrichtung zur Detektion von biologischen Signalen (1A, 1B) nach einem der Ansprüche 1 bis 3, wobei das körperseitig anliegende Polsterelement (20) mit einem dazwischen liegenden Rutschhemmer (16) auf dem Basispolsterelement (10) gestapelt ist.

5. Vorrichtung zur Detektion von biologischen Signalen (1A) nach einem der Ansprüche 1 bis 4, wobei der Sensor (30A, 30B, 30C) eine Vielzahl von Sensoren einschließt, die in gleichen Abständen an einem gleichen Umfang auf dem Basispolsterelement (10) vorgesehen sind.

6. Vorrichtung zur Detektion von biologischen Signalen (1A) nach einem der Ansprüche 1 bis 5, wobei eine umlaufende Nählinie (211) dadurch ausgebildet ist, dass ein Abschnitt in der Umfangsrichtung leicht nach außen von der Mitte weggenäht wird, um ein körperseitig anliegendes Netz (21) zusammenzudrücken, wobei der Sensor (30A, 30B, 30C, 30D) unter Verwendung eines Halters (31A, 31B, 31C, 31D) an einer Position angeordnet ist, die in einer Draufsicht einer weiter außen gelegenen Seite entspricht als die umlaufende Nählinie (211).

## Revendications

1. Dispositif (1A, 1B) de détection de signal biologique comprenant :
un élément d'amortissement (10) de base incluant : un treillis (11) côté base présentant un tissu tricoté tridimensionnel ; et un capteur (30A, 30B, 30C, 30D) disposé sur une surface ou une autre surface du treillis (11) côté base ; et
un élément d'amortissement (20) côté contigu au corps empilé sur l'élément d'amortissement (10) de base et incluant un treillis (21) côté contigu au corps qui présente un tissu tricoté tridimensionnel et est disposé sur un côté appuyant sur un corps d'une personne,
**caractérisé en ce que** l'élément d'amortissement (10) de base et l'élément d'amortissement (20) côté contigu au corps sont tous les deux sensiblement circulaires sur une vue en plan,
dans lequel l'élément d'amortissement (20) côté contigu au corps est empilé sur l'élément d'amortissement (10) de base avec un élément arbre (40) prévu entre des parties centrales de l'élément d'amortissement (20) côté contigu au corps et de l'élément d'amortissement (10) de base, et
dans lequel le capteur (30A, 30B, 30C, 30D) est disposé dans une plage dégagée de l'élément arbre (40).

2. Dispositif (1A, 1B) de détection de signal biologique selon la revendication 1, dans lequel un rebord circonférentiel de l'élément d'amortissement (20) côté contigu au corps est comprimé et recouvert d'un objet (22) en forme de bande.

3. Dispositif (1A, 1B) de détection de signal biologique selon la revendication 1 ou revendication 2, dans lequel l'élément d'amortissement (20) côté contigu au corps présente un diamètre supérieur ou égal au diamètre de l'élément d'amortissement (10) de base.

4. Dispositif (1A, 1B) de détection de signal biologique selon l'une quelconque des revendications 1 à 3, dans lequel l'élément d'amortissement (20) côté contigu au corps est empilé sur l'élément d'amortissement (10) de base avec un inhibiteur (16) de glissement entre eux.

5. Dispositif (1A) de détection de signal biologique selon l'une quelconque des revendications 1 à 4, dans lequel le capteur (30A, 30B, 30C) inclut une pluralité de capteurs prévus à intervalles égaux sur une même circonférence de l'élément d'amortissement (10) de base.

6. Dispositif (1A) de détection de signal biologique selon l'une quelconque des revendications 1 à 5, dans lequel une ligne de couture circonférentielle (211) est formée en cousant une partie légèrement écartée vers l'extérieur par rapport au centre dans la direction circonférentielle, pour comprimer un treillis (21) côté contigu au corps, dans lequel le capteur (30A, 30B, 30C, 30D) est disposé à l'aide d'un support (31A, 31B, 31C, 31D) à une position correspondant à un côté plus extérieur que la ligne de couture circonférentielle (211) sur une vue en plan.
